# EUROPEAN PATENT APPLICATION

(11) **EP 4 725 412 A1**
(43) Date of publication of application: **15.04.2026**
(21) Application number: 24206295.8
(22) Date of filing: 12.10.2024
(51) Int. Cl.: A61B 5/145, A61B 5/1455, A61B 5/00, A61B 5/1459

(54) **IMPLANT AND METHOD OF METABOLITE MEASUREMENT**

(71) Applicant: Walletmed Limited, Dublin D07 P4AX (IE)
(72) Inventor: BUSZYNSKI, Jakub, 60-452 Poznan (PL); GASIOR, Micha, 05-270 Marki (PL); MITURSKI, Andrzej, 21-003 Jakubowice Koninskie (PL); PAPROTA, Wojciech, 21-030 Motycz (PL)
(74) Representative: Korbela, Anna

(57) **Abstract**

The subject of the invention is a subcutaneous implant (1) for in vivo measurements of metabolites, consisting of an optically transparent housing (2) isolating (from the bodily fluids of a living organism) the filling in the form of:
- a reference system (3) and
- a mirror (4) made of a substance with high infrared reflectivity.

The method of in vivo metabolite measurement involves implanting the implant (1) - in the form of an optically transparent housing (2) with a filling in the form of a reference system (3) and a mirror (4) made of a substance with high infrared reflectivity - under the skin of a living organism, into the skin layer between the dermis and subcutaneous tissue, primarily in the area where there are capillaries between the epidermis and the mirror (4).

Next, when performing the measurement of metabolites, the implant (1) located in the optical path of a Raman spectrometry unit (5) or another optical spectrometry unit emitting infrared radiation is used.

The reference system (3) is a reference point for measuring metabolites, and the mirror (4) reflects the infrared radiation, which then returns to the Raman spectrometry unit (5) or another optical spectrometry unit. In the Raman spectrometry unit (5) or another optical spectrometry unit, spectra are normalized using the truth provided by the reference system (3). Then the normalized Raman spectra are compared with the Raman peak of the target molecule to measure the metabolite and determine the concentration of the metabolites in the living organism.

## Description

The subject of the invention is a subcutaneous implant for in vivo measurement of metabolites, including blood glucose or glucose in the subcutaneous space of a living organism, using a Raman spectrometry unit or another optical spectrometry unit.

The subject of the invention also includes a method for in vivo measurement of metabolites (found in the blood of a living organism) through blood vessels using a Raman spectrometry unit or another optical spectrometry unit.

Both the measurement technique using Raman spectrometry and the entire field of knowledge were developed based on the Raman effect. The phenomenon was theoretically predicted as early as 1923, but its empirical confirmation was found by the Indian physicist Chandrasekhara Raman in 1928. Since then, the knowledge of the Raman effect has been significantly expanded and systematized. Raman spectroscopy, like infrared (IR) spectroscopy, is a form of vibrational spectroscopy. It is based on the scattering of photons by particles. The Raman spectrum, however, arises due to a change in the polarization of the particle, rather than a dipole moment as in IR spectroscopy. Thus, Raman spectroscopy is based on the interaction of light with the chemical bonds of a substance, providing detailed information on chemical structure, polymorphism, crystallinity, and molecular dynamics.

The Raman spectrum is like a chemical fingerprint that identifies a molecule or material. Just like a human fingerprint, it can be compared to reference libraries to quickly identify or evaluate a material or distinguish it from others. Such spectral libraries of Raman spectra often contain hundreds of entries, with which the sample's spectrum is compared to determine the analyte. This provides insight, for example, into the chemical composition and properties of the tested sample.

Sensors using Raman spectroscopy are increasingly being applied where classical chemical substance control takes too long or is inefficient. Raman spectroscopy can also be used in all areas where non-destructive (microscopic) chemical analysis and imaging are required. It provides answers to both qualitative and quantitative analytical questions. In general, Raman spectroscopy is easy to use and quickly provides key information to characterize the chemical composition and structure of a sample. Essentially, it does not matter whether the samples are solid, liquid, or gaseous. Thus, Raman spectroscopy has found applications, for example, in industry, pharmaceuticals, geology and mineralogy, and medicine.

Raman spectroscopy is a universal technique and works for both inorganic and organic materials. However, it relies on a relatively weak Raman effect, and other spectroscopic effects and certain material properties can interfere. In the case of sample fluorescence, the sample will not provide a good Raman spectrum. Another, more significant problem includes strongly absorbing samples (e.g., water in the body).

Various methods of measurement using Raman spectrometry are therefore known.

For example, from the description of the American invention *"Apparatus for non-invasive in vivo measurement by Raman spectroscopy"* US10433775B2 with a priority date of 07.01.2010, a solution is known in which the metabolite concentration is measured in vivo by Raman spectroscopy in such a way that at the measurement site, at a depth of 200-300 µm below the skin surface, one receives light scattered from the metabolite present in the interstitial fluid of the skin, ensuring better retention of correct calibration and the possibility of transferring calibration between different subjects.

From the Chinese invention *"Use of coherent Raman techniques for medical diagnostic and therapeutic purposes, and calibration techniques for same"* CN101605494A, with a priority date of 27.10.2006, systems and methods are known that enable non-invasive, continuous, real-time molecular detection and quantitative determination of molecular species in a sample or an animal using Raman spectroscopy. Such systems and methods can be used to identify and quantify molecular species present in the body, which may be useful in prenatal diagnosis, detection of deep skin infections, evaluation of cerebrospinal fluid, measurement of arterial blood gases, blood glucose levels, cardiac biomarkers, creatinine clearance. Non-invasive, continuous, real-time quantitative assessment of such molecules enables a much more attractive course of therapy than existing protocols.

The European application *"Dielectrophoresis-based dynamic SERS nanoelement for classification and analysis of metabolites"* EP4317951A1, priority date 17.11.2021, is also known. It describes a dielectrophoresis-based dynamic nanoelement for surface-enhanced Raman spectroscopy (SERS) for the classification and analysis of metabolites. The method of analyzing metabolites using a dielectrophoresis-based dynamic SERS nanoelement may include: preparing a dynamic SERS nanoelement in which the surface region is formed as a matrix; applying a voltage to the dynamic SERS nanoelement; and classifying the metabolites of the analysis object by region by controlling the polarization of the dynamic SERS nanoelement according to the applied voltage.

Another American invention, *"Diagnostic marker compounds and their use"* US10080811B2, priority date 07.03.2012, is known, relating to monitoring biological substances, such as non-invasive monitoring of such substances in animals, for example biomarkers and metabolites. In particular, the invention relates to such monitoring using marker compounds labeled with rare earth elements. The invention also relates to such monitoring using laser spectroscopy or Raman spectroscopy, as well as the use of such monitoring in disease states such as stroke, neurological disorders, and cardiovascular disorders. Furthermore, the invention relates to new marker compounds conjugated with rare earth elements and the processes for producing these rare earth element-conjugated compounds.

The known solutions for measuring metabolites have most often consisted of pricking the fingertip to obtain a drop of blood for further testing, or involved subcutaneously implanting a spectrometer.

From the prior art, non-invasive methods for measuring metabolites in the form of determining blood glucose levels are also known.

From the description of the American invention *"Non-invasive determination of glucose"* US2022338765A1, a device for the non-invasive determination of blood glucose in a subject is known, which comprises:
a) a unit for receiving the examined area of the body from the subject,
b) a radiation source for generating terahertz radiation, in particular terahertz radiation in the wavelength range from about 0.1 mm to about 5 mm, from about 0.12 mm to about 5 mm or from about 0.1 mm to about 1 mm, for irradiating the examined area of the body,
c) a device for collecting radiation from the examined area of the body, consisting of:
   i) a device for collecting reflected terahertz radiation from the examined area of the body, which device is intended to collect terahertz radiation in the wavelength range in which the intensity of the reflected terahertz radiation changes depending on the glucose concentration,
   (ii) a device for collecting the body's own infrared radiation from the examined area of the body, which device is intended to separately collect infrared radiation at at least two different wavelengths or wavelength ranges in the wavelength range from about 8 µm to about 12 µm, at the first wavelength or the first wavelength range, where the intensity of the body's own infrared radiation is essentially independent of the glucose concentration, and at the second wavelength or second wavelength range where the intensity of the body's own infrared radiation changes depending on the glucose concentration, and wherein the device is optionally additionally designed to non-specifically collect the body's own infrared radiation,
d)
   (i) an element for measuring temperature in the examined area of the body,
   (ii) optionally an element for regulating the temperature in the examined area of the body,
e)
   (i) an element for measuring temperature in the collecting assembly (c),
   (ii) an element for regulating the temperature in the collecting assembly (c), so that the temperature in the collecting assembly (c) is lower than the temperature in the examined area of the body, and
f) a device designed to evaluate the signals from the collecting assembly (c) with temperature compensation and to determine the glucose concentration based on the evaluated signals, the device optionally also being adapted to selectively evaluate the body's own infrared radiation coming from the capillaries of the dermis of the examined area of the body.

From the prior art, a solution is also known in the form of a sensor placed on the skin on the user's arm. This sensor automatically measures and records sugar levels both day and night. The sensor has a needle that is inserted into the deeper layers of the skin, and the surface of its external part (the surface adhering to the user's skin) is covered with an adhesive substance. The mentioned sensor is waterproof and is about the size of a five-zloty coin. Glucose readings are automatically sent in real-time to a smartphone, where the current result and a trend arrow are visible, as well as a glycemic chart from the last 8 hours, created from measurements automatically recorded by the sensor. The sensor has optional alarms activated in the electronic application, so the user automatically receives a notification on the smartphone when their sugar level is too low or too high. However, since the sensor is placed on the body (and not subcutaneously), it is certainly visible and can be more easily damaged externally, as well as lead to skin irritation. Moreover, the sensor requires regular replacement (after a maximum of 14 days from placement on the skin).

The aim of the developed invention is to devise an economical method that will enable the most accurate possible measurement of glucose in the blood of a living organism, as well as the measurement of in vivo metabolites, allowing for an unambiguous measurement result.

At the same time, the aim of the developed invention is to simplify the process of measuring glucose or other metabolites so that this process is as fast as possible and does not require the use of additional external elements that until now were necessary to reflect Raman spectroscopy beams and thus obtain the measurement result.

Another aim of the developed invention is to shorten the optical path of the infrared radiation and thereby obtain more precise measurement results (even when using a weaker infrared radiation beam).

Another aim of the developed invention is to develop an implant that will be located under the skin, thereby not hindering the daily functioning of the living organism, and at the same time, not requiring replacement with a new implant.

The essence of the invention, which is a subcutaneous implant for in vivo measurement of metabolites, is that it is formed by an optically transparent housing that isolates from the bodily fluids of the living organism:
- a filling in the form of a reference system, and
- a mirror made of a substance with high infrared reflectivity.

Advantageously, the implant is shaped like a capsule, a disk that is concave or convex on one or both sides, or flat, a flattened capsule, or a cuboid with rounded corners.

Typically, the implant in the form of a capsule or a flattened capsule has a diameter at its widest point of from 0.5 mm to 5 mm and a length from 3 mm to 25 mm.

Most often, the implant in the form of a disk has a diameter from 3 mm to 25 mm and a height from 0.5 mm to 5 mm.

Advantageously, the dimensions of the sides of the implant in the form of a cuboid with rounded corners are from 0.5 mm to 25 mm, and the height from 0.5 mm to 25 mm, and the rounding of the corners is from 0.1 mm to 12.5 mm.

Often, the housing is made of sapphire, sapphire glass, borosilicate glass, quartz glass, soda-lime glass, aluminosilicate glass, lead glass, vycor glass.

Usually, the reference system of the implant consists of diamond nanoparticles ranging in size from 5 µm to 20 nm.

Advantageously, in the implant, the mirror forms an additional internal coating of the housing over all or part of the internal surface of the housing.

Most often, in the developed implant, the mirror is placed in the center of the housing, touching the housing only at the edges.

Usually, the surface of the mirror in the developed implant is flat, concave, convex, or shaped similarly to the implant.

Often, the mirror surface of the implant is made of silver, gold, titanium, chromium, or an alloy of these metals.

The essence of the developed method for measuring metabolites lies in the fact that the implant - in the form of an optically transparent housing with a filling in the form of a reference system and a mirror made of a substance with high infrared reflectivity - is implanted under the skin of a living organism into the skin layer between the dermis and subcutaneous tissue (primarily in the area where there are capillaries between the epidermis and the mirror). That is, the depth of the upper part of the implant is from 1 mm to 7 mm.

Then, to measure the metabolites, the implant located in the optical path of a Raman spectrometry unit or another optical spectrometry unit emitting infrared radiation is used. The infrared radiation passes through the implant's housing without any change in the properties of the radiation.

The reference system in the implant provides a reference point for metabolite measurement. The mirror reflects the infrared radiation, which then returns to the Raman spectrometry unit or other optical spectrometry unit.

In the Raman spectrometry unit or another optical spectrometry unit, the spectra are normalized using the truth provided by the reference system. Then, the normalized spectra are compared with the peak of the target molecule to measure the metabolite and determine the concentration of the metabolites in the living organism.

Usually, the implant is inserted in a well-perfused location, i.e., in the thigh, calf, lower abdomen, arm, wrist, the space between the fingers.

Advantageously, the implant is placed subcutaneously in the living organism for an unlimited period.

Most often, the Raman peak of the reference system is used to correct spectral shifts and intensity changes - including intensity normalization - in the Raman spectrometry unit or another optical spectroscopy method.

The advantage of the invention is the development of a solution and method for measuring metabolites that allow shortening the optical path by reducing the reflection distance and thus selecting the measurement volume (e.g., in the interstitial space just beneath the dermis), and thus obtaining an extremely reliable measurement result of metabolite levels in the human body. The developed solution and method also allow non-invasive real-time measurements.

Another advantage of the development is providing a reference system with diamond nanoparticles as a physical reference point, and additionally, the implant's housing is made of materials neutral to the living organism to exclude the risk of implant rejection or allergic reaction to the used materials.

The invention also allows reducing the problem of depth, scattering, and penetration width of the laser beam in the tissue (through the skin of the living organism), while at the same time returning to the Raman spectrometry unit even more than 50% of the infrared radiation (necessary to obtain the metabolite level reading) that the Raman spectrometry unit originally emitted. In addition to the Raman scattering signal, the developed solution also allows measurement of the absorption signal.

The subject of the invention is shown in the figure, where:

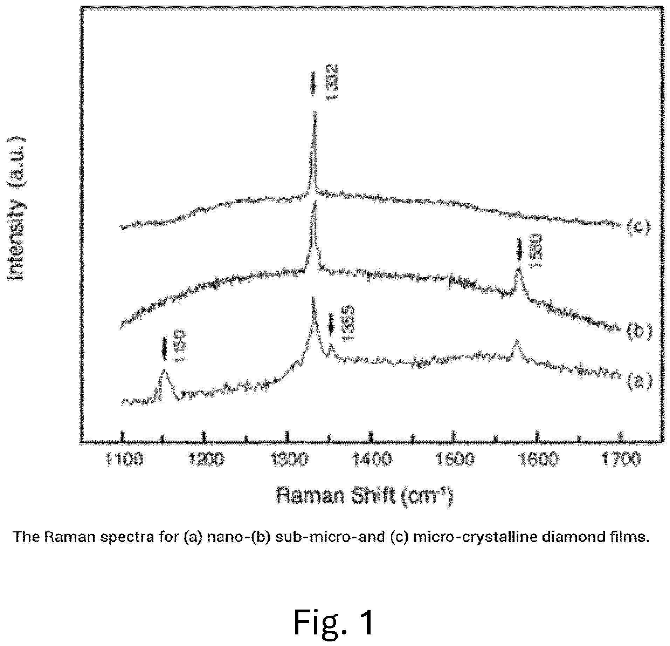

Fig. 1 - shows a graph depicting Raman spectra with Raman peaks of the target metabolite molecule after a purely optical measurement in a living organism,

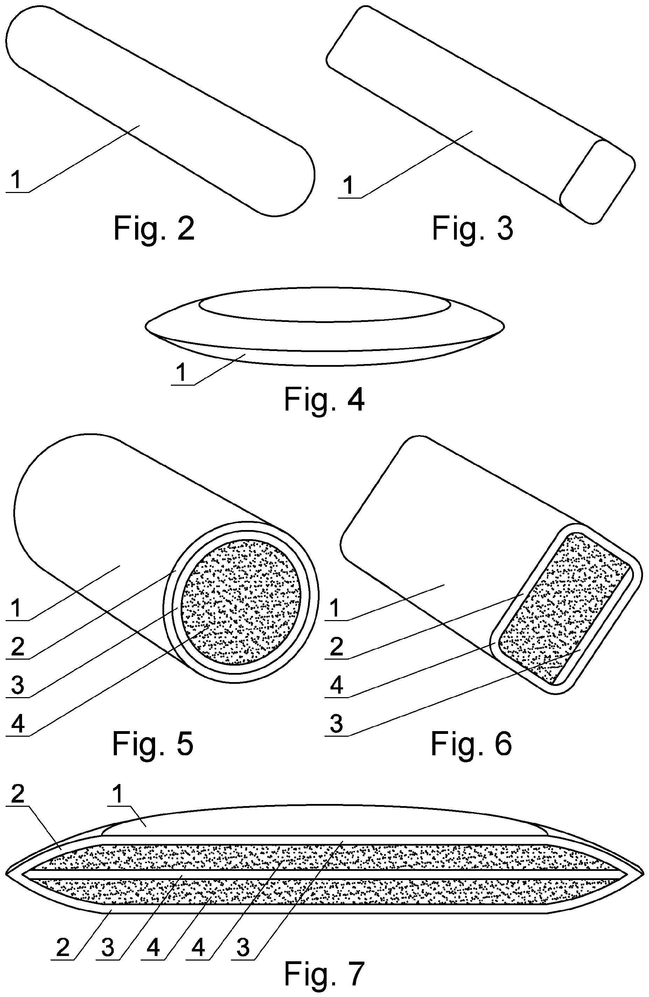

Fig. 2 - shows an implant in capsule shape,
Fig. 3 - shows an implant in flattened capsule shape,
Fig. 4 - shows an implant in disk shape,
Fig. 5 - shows a cross-section of a capsule-shaped implant with the capsule housing and the filling forming the reference system and mirror visible,
Fig. 6 - shows a cross-section of a flattened capsule-shaped implant with the capsule housing and the filling forming the reference system and mirror visible,
Fig. 7 - shows a cross-section of a disk-shaped implant with the capsule housing and the filling forming the reference system and mirror visible,

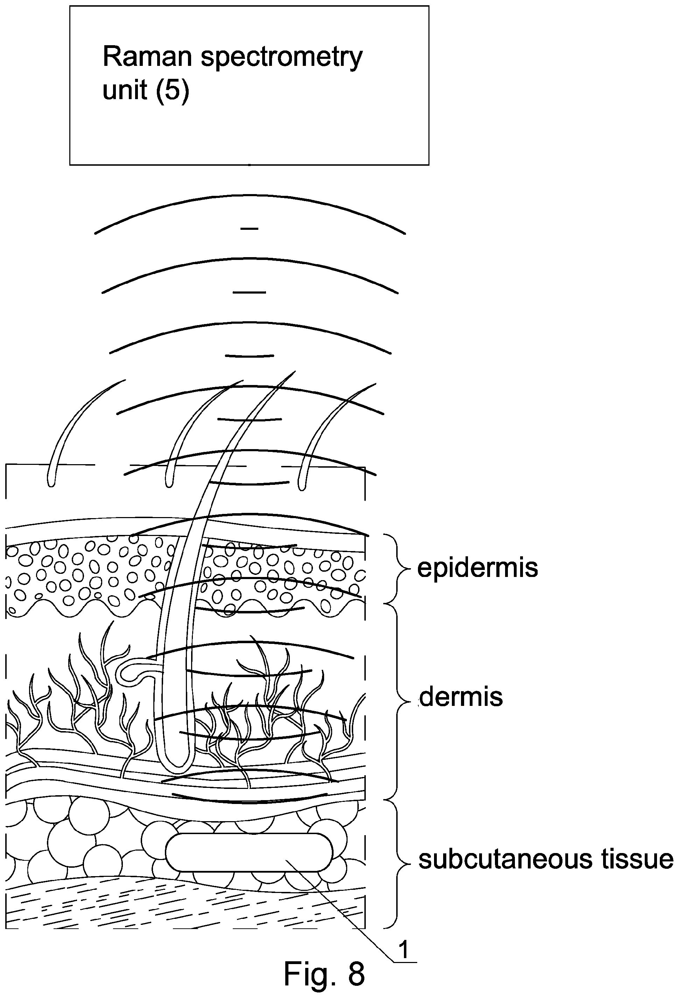

Fig. 8 - shows a capsule-shaped implant placed subcutaneously in a living organism.

As shown in the figures, the subject of the invention is an implant 1. The implant 1 is in the shape of a capsule, a disk (concave, convex, or flat on one or both sides), a flattened capsule, or a cuboid with rounded corners. The diameter of the implant 1 in the form of a capsule is from 0.5 mm to 5 mm and length from 3 mm to 25 mm (the implant 1 in the form of a flattened capsule has dimensions similar to that of a capsule-shaped implant 1). Meanwhile, the implant 1 in the form of a disk has a diameter from 3 mm to 25 mm and a height from 0.5 mm to 5 mm. The dimensions of the sides (length and width) of the implant 1 in the form of a cuboid with rounded corners are from 0.5 mm to 25 mm, and the height from 0.5 mm to 25 mm. The corner rounding of the implant 1 in the form of a cuboid with rounded corners ranges from 0.1 mm to 12.5 mm. The implant 1 is a housing 2 with a filling that is the reference system 3 and a mirror 4.

The housing 2 is an optical element (optically transparent), as the housing 2 is made of a material highly transparent to infrared radiation (long, medium, short), such as sapphire glass or borosilicate glass, quartz glass, soda-lime glass, aluminosilicate glass, lead glass, vycor glass, or metal (or other substances with similar physical and chemical properties), i.e., the radiation passes through the housing 2 of the implant 1 without affecting the radiation.

The housing 2 serves to isolate the filling in the form of the reference system 3, preferably diamond nanoparticles, as well as to isolate the mirror 4 from the bodily fluids of the living organism.

The reference system 3 contained in the housing 2 is a physical reference point in Raman spectrometry (or another optical spectrometry unit) for the measurement of metabolites. The reference system 3 preferably comprises diamond nanoparticles that have a size range suitable for effective Raman scattering, i.e., preferably from 5 µm (micrometers) to 20 nm (nanometers). The reference system 3 is a reference point for normalizing Raman spectra and appropriate calibration using the Raman peak to adjust data obtained from metabolites. The Raman peak of the reference system 3 is used for comparison with peaks of other molecules, such as glucose, for non-invasive in vivo metabolite measurements or for ex vivo measurements of biological samples.

The mirror 4 is placed inside the implant 1, i.e., inside the housing 2. The mirror 4 may form an additional internal coating of the housing 2 over the entire or a portion of the internal surface of the housing 2. The mirror 4 can also be placed in the center of the housing 2, touching it only at the edges. The surface of the mirror 4 can thus be flat, concave, convex, or shaped similarly to the implant 1. The mirror 4 is made of substances such as silver, gold, titanium, chromium, or an alloy of these metals, or other metals or substances with high infrared reflectivity (long, medium, short).

The method for measuring metabolites according to the developed invention involves implanting the described implant 1 under the skin of a living organism (human or animal) in the skin layer in the space between the dermis and subcutaneous tissue, primarily in an area where there are capillaries between the epidermis and the mirror 4. This means that the depth at which the upper part of the implant 1 is placed is from 1 mm to 7 mm. The implant 1 can be introduced anywhere in the living organism, preferably in a well-perfused area, and so that it is convenient for use and measurement. The implant 1 can be introduced into the thigh, calf, lower abdomen, arm, wrist, or the space between the fingers.

The implant 1 may remain in the living organism for an unlimited period of time, enabling measurements of metabolites during this period (at any desired frequency and amount). After a specified period, the implant 1 must be replaced with a new one.

Next, to measure the metabolites, the implant 1 (consisting of the housing 2 and the reference system 3 and mirror 4 inside the housing 2) is used, and is located in the optical path of a Raman spectrometry unit 5 or another optical spectrometry unit emitting infrared radiation. The infrared radiation passes through the housing 2 of the implant 1 without changing the properties of the radiation, because the housing 2 is made of a material highly transparent to infrared radiation (long, medium, short), such as sapphire glass, borosilicate glass, quartz glass, soda-lime glass, aluminosilicate glass, lead glass, vycor glass (or other substances with similar physical and chemical properties).

The reference system 3 (preferably in the form of diamond nanoparticles) provides a reference point for metabolite measurements. The mirror 4 reflects the infrared radiation, which then returns to the Raman spectrometry unit 5 or another optical spectrometry unit. In the Raman spectrometry unit 5, Raman spectra are normalized using the truth provided by the reference system 3. Then the normalized Raman spectra are compared with the Raman peak of the target molecule, such as glucose or other metabolites, to measure the metabolite and determine its concentration in the living organism. The Raman peak of the reference system 3 can be used to correct spectral shifts and intensity changes in the Raman spectrometry unit 5.

Metabolite measurement using the Raman spectrometry unit 5 or another optical spectrometry unit can be performed as non-invasive in vivo measurements of metabolites or as ex vivo measurements of biological samples.

### List of reference signs:

- 1.: implant,
- 2.: housing,
- 3.: reference system,
- 4.: mirror,
- 5.: Raman spectrometry unit

## Claims

1. A subcutaneous implant (1) for in vivo measurement of metabolites, **characterized in that** it comprises an optically transparent housing (2) that isolates from the bodily fluids of a living organism:
- a filling in the form of a reference system (3) and
- a mirror (4) made of a substance with high infrared reflectivity.

2. The implant (1) according to claim 1, **characterized in that** it is shaped like a capsule, a disk that is concave or convex on one or both sides, or flat, a flattened capsule, or a cuboid with rounded corners.

3. The implant (1) according to claim 1 or 2, **characterized in that** the diameter of the implant (1) in the form of a capsule or a flattened capsule is from 0.5 mm to 5 mm at its widest point, and it has a length from 3 mm to 25 mm.

4. The implant (1) according to claim 1 or 2, **characterized in that** the implant (1) in the form of a disk has a diameter from 3 mm to 25 mm and a height from 0.5 mm to 5 mm.

5. The implant (1) according to claim 1 or 2, **characterized in that** the dimensions of the sides of the implant (1) in the form of a cuboid with rounded corners are from 0.5 mm to 25 mm, and the height from 0.5 mm to 25 mm, and the corner rounding is from 0.1 mm to 12.5 mm.

6. The implant (1) according to any of claims 1 to 5, **characterized in that** the housing (2) is made of sapphire, or sapphire glass, borosilicate glass, quartz glass, soda-lime glass, aluminosilicate glass, lead glass, vycor glass.

7. The implant (1) according to any of claims 1 to 6, **characterized in that** the reference system (3) consists of diamond nanoparticles ranging in size from 5 µm to 20 nm.

8. The implant (1) according to any of claims 1 to 7, **characterized in that** the mirror (4) forms an additional internal coating of the housing (2) over all or part of the internal surface of the housing (2).

9. The implant (1) according to any of claims 1 to 7, **characterized in that** the mirror (4) is placed in the center of the housing (2), touching the housing (2) only at the edges.

10. The implant (1) according to any of claims 1 to 9, **characterized in that** the surface of the mirror (4) is flat, concave, convex, or shaped like the implant (1).

11. The implant (1) according to any of claims 1 to 10, **characterized in that** the surface of the mirror (4) is made of silver, gold, titanium, chromium, or an alloy of these metals.

12. A method of measuring metabolites, **characterized in that** the implant (1) - in the form of an optically transparent housing (2) with a filling in the form of a reference system (3) and a mirror (4) made of a substance with high infrared reflectivity - is implanted under the skin of a living organism into the skin layer in the space of the dermis and subcutaneous tissue, primarily in the area where there are capillaries between the epidermis and the mirror (4), meaning that the depth at which the upper part of the implant (1) is placed is from 1 mm to 7 mm,
then, when performing the measurement of metabolites, the implant (1) located in the optical path of a Raman spectrometry unit (5) or another optical spectrometry unit emitting infrared radiation is used, the infrared radiation passes through the housing (2) of the implant (1) without changing the radiation properties,
the reference system (3) is a reference point for metabolite measurement, and the mirror (4) reflects the infrared radiation, which then returns to the Raman spectrometry unit (5) or another optical spectrometry unit,
in the Raman spectrometry unit (5) or another optical spectrometry unit, spectral normalization is carried out using the truth provided by the reference system (3), then the normalized spectra are compared with the peak of the target molecule.

13. The method of measuring metabolites according to claim 12, **characterized in that** the implant (1) is introduced at a well-perfused site, i.e., in the thigh, calf, lower abdomen, arm, wrist, or the space between the fingers.

14. The method of measuring metabolites according to claim 12 or 13, **characterized in that** the implant (1) is placed subcutaneously in the living organism for an unlimited period of time.

15. The method of measuring metabolites according to claim 12, 13, or 14, **characterized in that** the Raman peak of the reference system (3) is used to correct spectral shifts and intensity changes - including intensity normalization - in the Raman spectrometry unit (5) or another optical spectroscopy method.
